# EUROPÄISCHE PATENTSCHRIFT

(11) **EP 2 934 471 B1**
(45) Veröffentlichungstag und Bekanntmachung des Hinweises auf die Patenterteilung: **13.09.2017**
(21) Anmeldenummer: 13789544.7
(22) Anmeldetag: 11.11.2013
(51) Int. Cl.: A61K 8/49, A61K 8/42, A61Q 5/06

(54) **VERFAHREN ZUR SCHONENDEN WÄRMEUNTERSTÜTZTEN UMFORMUNG KERATINHALTIGER FASERN**
METHOD FOR THE GENTLE HEAT-ASSISTED SHAPING OF KERATIN FIBERS
PROCÉDÉ DE MISE EN FORME THERMIQUE NON AGRESSIVE DE FIBRES KÉRATINIQUES

(30) Priorität: 21.12.2012 DE 102012224143
(43) Veröffentlichungstag der Anmeldung: 28.10.2015
(73) Patentinhaber: CRODA INTERNATIONAL PLC, Goole East Yorkshire DN14 9AA (GB)
(72) Erfinder: METTEN, Diane, 22393 Hamburg (DE); RICHTERS, Bernd, 21129 Hamburg (DE); SCHEFFLER, Rene, 25479 Ellerau (DE)
(74) Vertreter: Best, Rachel Elizabeth
(86) Internationale Anmeldenummer: PCT/EP2013/073472
(87) Internationale Veröffentlichungsnummer: WO 2014/095158

(56) Entgegenhaltungen:
- WO-A1-01/97760
- WO-A1-87/06826
- WO-A1-98/51265
- DE-A1-102008 031 700
- JP-A- 2010 189 309
- US-A- 2 647 125
- DATABASE GNPD [Online] MINTEL; 31. März 2006 (2006-03-31), "Heat Protection Styling Spray", XP002732406, Database accession no. 10255740

## Beschreibung

Die Erfindung betrifft das technische Gebiet der Formgebung für keratinhaltige Fasern.
Als keratinhaltige Fasern können prinzipiell alle tierischen Haare, z.B. Wolle, Roßhaar, Angorahaar, Pelze, Federn und daraus gefertigte Produkte oder Textilien eingesetzt werden. Bevorzugt wird die Erfindung jedoch im Rahmen einer wärmeunterstützten Haarumformung, insbesondere der Glättung krauser menschlicher Haare und daraus gefertigter Perücken, eingesetzt.

Eine Verformung keratinhaltiger Fasern wird üblicherweise derart durchgeführt, dass man die Faser mechanisch verformt und die Verformung durch geeignete Hilfsmittel festlegt. Vor und/oder nach dieser Verformung behandelt man die Faser mit mindestens einer kosmetischen Zubereitung, um die dem Faserkollektiv neu aufgeprägte Form zu fixieren.

Im Rahmen einer Dauerwelle beispielsweise, werden keratinhaltige Fasen mit keratinreduzierenden Verbindungen behandelt. Nach einem Spülvorgang wird die Faser dann in dem so genannten Fixierschritt mit einer Oxidationsmittelzubereitung behandelt, gespült und nach oder während des Fixierschritts von den Verformungshilfsmitteln (z.B. Wicklern, Papilloten) befreit. Wenn als keratinreduzierende Komponente ein Merkaptan, z.B. Ammoniumthioglykolat, verwendet wird, spaltet dieses einen Teil der Disulfid-Brücken des Keratin-Moleküls zu -SH-Gruppen, so dass es zu einer Erweichung der Keratinfaser kommt. Bei der späteren oxidativen Fixierung werden erneut Disulfid-Brücken im Haarkeratin geknüpft, so dass das Keratingefüge in der vorgegebenen Verformung fixiert wird.

Im Rahmen einer temporären Formgebung wird die neu aufgeprägte Form des Faserkollektivs durch die Applikation von sogenannten Haarfixativen fixiert. Solche Haarfixative sind beispielsweise Wachse oder festigende Polymere.

Oftmals werden bei Einsatz wasserhaltiger Kosmetika die feuchten keratinhaltigen Fasern umgeformt, die Form zunächst mechanisch fixiert, um dann durch Anwendung von Wärme das Wasser zu entfernen und zusätzlich durch die Neuformung der Wasserstoffbrückenbindungen innerhalb und zwischen den Keratinproteinsträngen eine Fixierung der neuen Form zu bewerkstelligen.

Zur Glättung keratinhaltiger Fasern wird in einem entsprechenden Verfahren das krause Haar entweder auf Wickler mit einem großen Durchmesser von üblicherweise mehr als 15 mm gewickelt oder das Haar unter Einwirkung z.B ein keratinreduzierenden Zusammensetzung glattgekämmt. Anstelle des Wicklers ist es auch möglich, die Faser auf ein Glättungsboard glattzulegen. Glättungsboarde sind üblicherweise rechteckige Tafeln z.B. aus Kunststoff. Vorzugsweise ist die Faser dabei mit einer Flüssigkeit benetzt. Unterstützend werden die mechanisch in der neuen Form fixierten Fasern einer Wärmebehandung, beispielsweise durch ein heißes Gebläse oder dem Kontakt mit heißen Oberflächen, unterworfen.

Eine Möglichkeit der Haarglättung ist das Glätten mit einem heißen Eisen. Allerdings verändert sich die Struktur der keratinhaltigen Faser bei der Wärmebehandlung des Haars während des Glättens (siehe dazu R. McMullen et al., J. Cosmet. Sci., 1998, 49, 223-244). Dieser Änderung der Faserstruktur sollte durch geeignete Maßnahmen entgegengewirkt werden oder im Nachhinein repariert werden.

Aufgabe der Erfindung ist es daher, ein wärmeunterstütztes Umformungsverfahren für keratinhaltige Fasern, insbesondere für menschliches Haar, bereitzustellen, das eine sehr gute Formfixierung liefert, die Faser pflegt sowie die Struktur der Faser schont oder repariert.

Überraschenderweise wurde gefunden, dass die Aufgabe durch ein Umformungsverfahren für keratinhaltige Fasern, insbesondere menschliche Haare, gewährleistet wird, welches nach der Hitzeapplikation einen Nachbehandlungsschritt mit einem speziellen kosmetischen Wirkstoff Siehe in diesem Zusammenhang auch WO01/97760, WO87/06826 und DE102008032700. Ein erster Gegenstand der Erfindung ist daher die Verwendung von mindestens einer Verbindung der Formel (I) 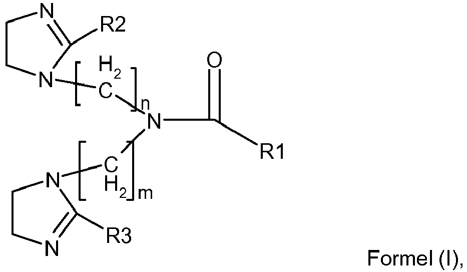 in welcher
- R1, R2 und R3 unabhängig voneinander stehen für einen aliphatischen Kohlenwasserstoffrest mit 6 bis 30 Kohlenstoffatomen, und
- n und m stehen unabhängig voneinander für ganze Zahlen von 1 bis 10, in einem Nachbehandlungsschritt nach der Anwendung von Wärme zur Restrukturierung keratinhaltiger Fasern, die Wärme von einer Temperatur von 80°C bis 350°C (vorzugsweise 80°C bis 280°C, besonders bevorzugt 100°C bis 250°C, weiter bevorzugt 140°C bis 220°C) ausgesetzt wurden. Als Wärmequelle dient bevorzugt ein Heißluftgebläse (beispielsweise in der Ausführungsform als Föhn, Haube oder Lockenstab), ein Glätteisen oder ein Welleisen. Glätteisen oder Welleisen sind besonders bevorzugte Wärmequellen.

Der besagte aliphatische Kohlenwasserstoffrest der Reste R1, R2 und R3 gemäß obiger Formel (I) kann linear oder verzweigt sein.

Der besagte aliphatische Kohlenwasserstoffrest der Reste R1, R2 und R3 gemäß obiger Formel (I) steht bevorzugt für eine C6-C30-Alkylgruppe oder eine C6-C30-Alkenylgruppe (besonders bevorzugt für eine C8-C24-Alkylgruppe oder eine C8-C24-Alkenylgruppe, ganz besonders bevorzugt für eine C10-C20-Alkylgruppe oder eine C10-C20-Alkenylgruppe, und höchst bevorzugt für eine C12-C18-Alkylgruppe oder eine C12-C18-Alkenylgruppe).

Es ist erfindungsgemäß bevorzugt, wenn zumindest die Reste R2 und R3 der Formel (I) gleich sind. Höchst bevorzugt sind alle Reste R1 und R2 und R3 gleich (R1 gleich R2 gleich R3).

Es ist erfindungsgemäß bevorzugt, wenn gemäß Formel (I) n = m ist.

Es ist erfindungsgemäß bevorzugt, wenn gemäß obiger Formel (I) n und m für ganze Zahlen von 2 bis 6 stehen und höchst bevorzugt für 2, 3 und/oder 4, wobei wiederum bevorzugt n = m ist.

Gemäß obiger Formel (I) stehen R1 bis R3 vorzugsweise für Capryl, Caprylyl, Octyl, Nonyl, Decanyl, Lauryl, Myristyl, Cetyl, Stearyl, Isostearyl, Oleyl, Behenyl oder Arachidyl. Weiterhin gilt wiederum besonders bevorzugt R2 gleich R3 und höchst bevorzugt R1 gleich R2 gleich R3. Die Buchstaben n und m stehen unabhängig voneinander für ganze Zahlen von 1 bis 10, vorzugsweise 2 bis 6 und höchst bevorzugt für 2, 3 und/oder 4, wobei höchst bevorzugt n = m ist.

Gemäß obiger Formel (I) sind höchst bevorzugt R1 gleich R2 gleich R3 und ausgewählt aus Capryl, Caprylyl, Octyl, Nonyl, Decanyl, Lauryl, Myristyl, Cetyl, Stearyl, Isostearyl, Oleyl, Behenyl oder Arachidyl und n = m = 2. Am bevorzugtesten ist R1 = R2 = R3 und ausgewählt aus Lauryl, Myristyl, Cetyl, Stearyl, Isostearyl, Oleyl, Behenyl oder Arachidyl, worunter Cetyl, Stearyl, Isostearyl, Oleyl oder Behenyl besonders bevorzugt sind und n = m = 2.

Die bevorzugteste Verbindung der Formel (I) ist diejenige, welche den INCI Namen Bis-Ethyl(isostearylimidazoline) Isostearamide trägt. Letztere Verbindung ist unter der Handelsbezeichnung Keradyn® HH von der Firma Croda im Handel erhältlich.

Ganz besonders bevorzugt wird besagte Verbindung der obigen Formel (I) im Rahmen einer Restrukturierung keratinhaltiger Fasern, insbesondere menschlicher Haare, die zuvor einer wärmeunterstützten Glättung bei einer Temperatur von 80°C bis 350°C (vorzugsweise 80°C bis 280°C, besonders bevorzugt 100°C bis 250°C, weiter bevorzugt 140°C bis 220°C) unterworfen wurden, verwendet.

Im Rahmen der erfindungsgemäßen Verwendung hat es sich als günstig und daher erfindungsgemäß bevorzugt herausgestellt, mindestens eine Verbindung der obigen Formel (I) in einen kosmetischen Träger einzuarbeiten und das resultierende kosmetische Mittel zur Nachbehandlung der Fasern im Rahmen einer wärmeunterstützten Umformung zu verwenden.

Die erfindungsgemäß verwendeten Mittel enthalten die Verbindungen der obigen Formel (I) bevorzugt in einer Menge von 0,01 bis 15,0 Gew.-%, besonders bevorzugt von 0,1 bis 10,0 Gew.-%, ganz besonders bevorzugt von 0,1 bis 7,5 Gew.-%, höchst bevorzugt von 0,3 bis 5,0 Gew.% jeweils bezogen auf das Gewicht des anwendungsbereiten Mittels.

Im Rahmen einer Ausführungsform der Erfindung ist es bevorzugt, dass das erfindungsgemäß verwendete Mittel zusätzlich mindestens ein festigendes Polymer enthält, ausgewählt aus mindestens einer Verbindung aus der Gruppe, die gebildet wird aus festigenden nichtionischen Polymeren, festigenden anionischen Polymeren, festigenden amphoteren Polymeren und festigenden kationischen Polymeren.
Zusätzlich enthält das erfindungsgemäß verwendete Mittel bevorzugt mindestens ein festigendes kationisches Polymer enthalten. Die zusätzlichen festigenden kationischen Polymere weisen mindestens eine Struktureinheit auf, die mindestens ein permanent kationisiertes Stichstoffatom enthält. Unter permanent kationisierten Stickstoffatomen sind solche Stickstoffatome zu verstehen, die eine positive Ladung tragen und dadurch eine quartäre Ammoniumverbindung bilden. Quartäre Ammonium-Verbindungen werden meist durch Umsetzung tertiärer Amine mit Alkylierungsmitteln, wie z.B. Methylchlorid, Benzylchlorid, Dimethylsulfat, Dodecylbromid, aber auch Ethylenoxid hergestellt. In Abhängigkeit von dem eingesetzten tertiären Amin sind insbesondere folgende Gruppen bekannt: Alkylammonium-Verbindungen, Alkenylammonium-Verbindungen, Imidazolinium-Verbindungen und Pyridinium-Verbindungen.
Im Sinne dieser Ausführungsform bevorzugte Mittel enthalten die festigenden kationischen Polymere in einer Menge von 0,1 Gew.-% bis 20,0 Gew.-%, besonders bevorzugt von 0,2 Gew.-% bis 10,0 Gew.-%, ganz besonders bevorzugt von 0,5 Gew.-% bis 5,0 Gew.-%, jeweils bezogen auf das Gewicht des Mittels.

Die kationischen festigenden Polymere können erfindungsgemäß aus kationischen, quaternisierten Cellulose-Derivaten ausgewählt werden.

Es erweisen sich generell solche kationischen, quaternisierten Cellulosen als im Sinne der Ausführungsform vorteilhaft, die in einer Seitenkette mehr als eine permanente kationische Ladung tragen. Darunter sind unter den kationischen Cellulosederivaten solche hervorzuheben, die aus Reaktion von Hydroxyethylcellulose mit einem Dimethyldiallylammonium-Reaktanden (insbesondere Dimethyldiallylammoniumchlorid) gegebenenfalls in Gegenwart weiterer Reaktanden hergestellt werden. Unter diesen kationischen Cellulosen sind wiederum solche kationischen Cellulosen mit der INCI-Bezeichnung Polyquaternium-4 besonders geeignet.

Weiterhin eignen sich solche kationischen festigenden Polymere, die mindestens eine Struktureinheit der Formel (N1) und mindestens eine Struktureinheit der Formel (N2) und gegebenenfalls mindestens eine Struktureinheit der Formel (K1) umfassen 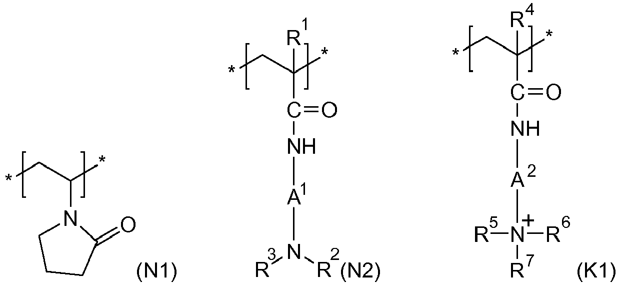 worin
R¹ und R⁴ stehen unabhängig voneinander für ein Wasserstoffatom oder eine Methylgruppe,
A¹ und A² stehen unabhängig voneinander für eine Gruppe Ethan-1,2-diyl, Propan-1,3-diyl oder Butan-1,4-diyl,
R² , R³, R⁵ und R⁶ stehen unabhängig voneinander für eine (C₁ bis C₄)-Alkylgruppe,
R⁷ steht für eine (C₈ bis C₃₀)-Alkylgruppe.

Geeignete Verbindungen sind beispielsweise als
- Copolymere aus mit Diethylsulfat quaterniertem Dimethylaminoethylmethacrylat, mit N-Vinylpyrrolidon mit der INCI-Bezeichnung Polyquaternium-11,
- Copolymere aus Methacryloylaminopropyllauryldimethylammonium chlorid mit N-Vinylpyrrolidon und Dimethylaminopropylmethacrylamid mit der INCI-Bezeichnung Polyquaternium-55,
- Copolymere aus Methacryloylaminopropyllauryldimethylammonium chlorid mit N-Vinylpyrrolidon, N-Vinylcaprolactam und Dimethylaminopropylmethacrylamid mit der INCI-Bezeichnung Polyquaternium-69.

Als im Sinne der Ausführungsform besonders bevorzugt verwendbare festigende kationische Polymere dienen weiterhin solche festigenden kationischen Copolymere, die ein Copolymer (c1) enthalten ist, das neben mindestens einem Strukturelement der Formel (K2) zusätzlich ein Strukturelement der Formel (N1) umfasst 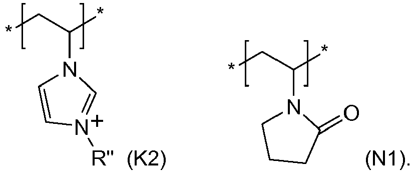 worin
R für eine (C₁ bis C₄)-Alkylgruppe, insbesondere eine Methylgruppe, steht.

Besonders bevorzugte N-Methylvinylimidazol/Vinylpyrrolidon-Copolymere werden laut INCI-Nomenklatur als Polyquaternium-16 bezeichnet. Die bevorzugten N-Methylvinylimidazol/Vinylpyrrolidon-Copolymere werden laut INCI-Nomenklatur als Polyquaternium-44 bezeichnet.
Weiterhin bevorzugt werden N-Methylvinylimidazol/Vinylpyrrolidon/Vinylcaprolactam-Copolymere, die laut INCI-Nomenklatur als Polyquarternium-46 bezeichnet werden.
Bevorzugt werden weiterhin N-Methylvinylimidazol/Vinylpyrrolidon/Vinylimidazol/Methacrylamid-Copolymere, die laut INCI-Nomenklatur als Polyquaternium-68 bezeichnet werden.

Ganz besonders bevorzugte Copolymere (c3) enthalten 1 bis 12 Mol-%, vorzugsweise 3 bis 9 Mol.-% und insbesondere 6 Mol.-% Struktureinheiten gemäß Formel (K2-a) und 45 bis 65 Mol.-%, vorzugsweise 50 bis 60 Mol.-% und insbesondere 55 Mol.-% Struktureinheiten gemäß Formel (N1) und 1 bis 20 Mol.-%, vorzugsweise 5 bis 15 Mol.-% und insbesondere 10 Mol.-% Struktureinheiten gemäß Formel (N3) und 20 bis 40 Mol.-%, vorzugsweise 25 bis 35 Mol.-% und insbesondere 29 Mol.-% Struktureinheiten gemäß Formel (N4).

Unter den zusätzlichen festigenden kationischen Polymer ausgewählt aus den kationischen Polymeren mit mindesten einem Strukturelement der obigen Formel (K2), gelten als bevorzugt:
- N-Vinylpyrrolidon/1-Vinyl-3-methyl-1H-imidazoliumchlorid-Copolymere,
- N-Vinylpyrrolidon/1-Vinyl-3-methyl-1H-imidazoliummethylsulfat-Copolymere,
- N-Vinylpyrrolidon/N-Vinylcaprolactam/1-Vinyl-3-methyl-1H-imidazolium-Terpolymer,
- N-Vinylpyrrolidon/Methacrylamid/N-Vinylimidazol/1-Vinyl-3-methyl-1H-imidazoliummethylsulfat-Copolymer,
sowie Gemische aus diesen Polymeren.

Das erfindungsgemäße Mittel enthält bevorzugt als zusätzliches festigendes Polymer mindestens ein festigendes nichtionisches Polymer. Unter einem nichtionischen Polymer wird erfindungsgemäß ein Polymer verstanden, das in einem protischen Lösemittel bei Standardbedingungen im Wesentlichen keine Struktureinheiten mit permanent kationischen oder anionischen Gruppen trägt, welche durch Gegenionen unter Erhaltung der Elektroneutralität kompensiert werden müssen. Die festigenden nichtionischen Polymere sind in dem erfindungsgemäßen Mittel dieser Ausführungsform bevorzugt in einer Menge von 0,1 Gew.-% bis 20,0 Gew.-%, besonders bevorzugt von 0,2 Gew.-% bis 15,0 Gew.-%, ganz besonders bevorzugt von 0,5 Gew.-% bis 10,0 Gew.-%, jeweils bezogen auf das Gewicht des erfindungsgemäßen Mittels, enthalten.

Die erfindungsgemäßen Mittel enthalten besonders bevorzugt als nichtionisches festigendes Polymer mindestens ein Chitosan. Neben den Chitosanen als typische Biopolymere kommen im Sinne der Erfindung als Derivate alkoxylierte Chitosane in Frage. Erfindungsgemäß bevorzugte Mittel sind dadurch gekennzeichnet, dass sie als Chitosan mindestens ein Neutralisationsprodukt von Chitosan mit mindestens einer organischen Carbonsäure enthalten. Hierbei ist es erfindungsgemäß bevorzugt, die organische Carbonsäure unter Milchsäure, Pyrrolidoncarbonsäure, Nicotinsäure, Hydroxyisobuttersäure, Hydroxyisovaleriansäure oder Gemischen dieser Säuren auszuwählen. Die Chitosane bzw. deren Derivate sind in den erfindungsgemäßen Mitteln bevorzugt in einer Menge von 0,01 Gew.-% bis 5 Gew.-%, besonders bevorzugt von 0,05 Gew.-% bis 2,0 Gew.-%, ganz besonders bevorzugt von 0,1 Gew.-% bis 1 Gew.-%, jeweils bezogen auf das Gewicht des erfindungsgemäßen Mittels, enthalten.

Die festigenden nichtionischen Polymere werden bevorzugt ausgewählt aus mindestens einem Polymer der Gruppe, die gebildet wird, aus
- Homopolymeren und nichtionischen Copolymeren des N-Vinylpyrrolidons,
- Homopolymeren und nichtionischen Copolymeren des Vinylalkohols,
- nichtionischen Copolymeren des Isobutens.

Mittel, die als festigendes nichtionisches Polymer mindestens ein Polymer ausgewählt aus der Gruppe, die gebildet wird aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymere aus N-Vinylpyrrolidon und N-Vinylimidazol und Methacrylamid,
- Copolymere aus N-Vinylpyrrolidon und N-Vinylimidazol und Acrylamid,
- Copolymere aus N-Vinylpyrrolidon mit N,N-Di(C₁ bis C₄)-Alkylamino-(C₂ bis C₄)-alkylacrylamid,
- Copolymere aus N-Vinylpyrrolidon mit N,N-Di(C₁ bis C₄)-Alkylamino-(C₂ bis C₄)-alkylacrylamid, sind erfindungsgemäß ganz besonders bevorzugt.

Mittel, die als festigendes nichtionisches Polymer mindestens ein Polymer ausgewählt aus der Gruppe, die gebildet wird aus N-Vinylpyrrolidon und Vinylacetat, sind erfindungsgemäß am bevorzugtesten.

Die erfindungsgemäßen Mittel können als festigendes Polymer auch mindestens ein festigendes amphoteres Polymer enthalten. Ein Beispiel für ein erfindungsgemäß einsetzbares festigendes amphoteres Polymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Alkylestern darstellt. Die festigenden amphoteren Polymere sind in den erfindungsgemäß einsetzbaren wasserbasierten Zusammensetzungen bevorzugt in Mengen von 0,01 Gew.-% bis 20 Gew.-%, besonders bevorzugt von 0,05 bis 10 Gew.-%, bezogen auf das gesamte Mittel, enthalten. Mengen von 0,1 bis 5 Gew.-% sind ganz besonders bevorzugt.

Weiterhin können als festigende Polymere mindestens ein festigendes anionisches Polymer eingesetzt werden. Wenn das erfindungsgemäße Mittel ein festigendes anionisches Polymer enthält, dann liegt sie bevorzugter Weise in Form eines Sprays, insbesondere eines Aerosolsprays, vor. Die festigenden anionischen Polymere sind in dem erfindungsgemäßen Mittel bevorzugt in einer Menge von 0,1 Gew.-% bis 20,0 Gew.-%, besonders bevorzugt von 0,2 Gew.-% bis 15,0 Gew.-%, ganz besonders bevorzugt von 0,5 Gew.-% bis 10,0 Gew.-%, jeweils bezogen auf das gesamte Mittels, enthalten.
Besonders bevorzugte Polymere dieser Art werden ausgewählt aus mindestens einem Polymer der Gruppe, die gebildet wird aus
- Copolymeren aus Vinylacetat und Crotonsäure.
- Copolymeren aus Vinylpropionat und Crotonsäure,
- Copolymeren aus Vinylneodecanoat, Vinylacetat und Crotonsäure.

Besonders bevorzugte Polymere werden ausgewählt aus mindestens einem Polymer der Gruppe, die gebildet wird aus Copolymeren aus Acrylsäure und Ethylacrylat und N-tert.-Butylacrylamid.
Darüber hinaus kann die erfindungsgemäß einsetzbare wasserbasierte Zusammensetzung als anionisches festigendes Polymer mindestens ein Polyurethan mit mindestens einer Carboxylgruppe enthalten (wie beispielsweise ein Copolymer aus Isophthalsäure, Adipinsäure, 1,6-Hexandiol, Neopentylglykol und).
Die festigenden anionischen Polymere sind bevorzugt in einer Menge von 0,1 Gew.-% bis 15 Gew.-%, insbesondere von 0,5 Gew.-% bis 10 Gew.-%, jeweils bezogen auf das Gewicht der wasserbasierten Zusammensetzung, enthalten.

Es hat sich als besonders bevorzugt erwiesen, wenn die erfindungsgemäßen Mittel zusätzlich mindestens ein kationisches Tensid enthalten. Dabei sind wiederum kationische Tenside ausgewählt unter quartären Ammoniumverbindungen, Esterquats und Amidoaminen bzw. Gemischen daraus bevorzugt. Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Es hat sich als besonders bevorzugt erwiesen, wenn die erfindungsgemäßen Mittel zusätzlich mindestens ein nichtionisches Tensid enthalten.
Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 100 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder (C₂ bis C₆)-Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester,
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R₄O-[G]p (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Als ganz besonders bevorzugte nichtionische Tenside haben sich die Alkylenoxid-Anlagerungsprodukte an gesättigte lineare Fettalkohole und Fettsäuren mit jeweils 2 bis 100 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside C₁₂-C₃₀-Fettsäuremono- und -diester von Anlagerungsprodukten von 1 bis 30 Mol Ethylenoxid an Glycerin und/oder Anlagerungsprodukte von 5 bis 60 Mol Ethylenoxid an Rizinusöl und gehärtetes Rizinusöl enthalten.
Ganz besonders bevorzugt enthalten die erfindungsgemäßen Mittel als Tensid mindestens ein Anlagerungsprodukt von 15 bis 100 mol Ethylenoxid, insbesondere von 15 bis 50 mol Ethylenoxid an einen linearen oder verzweigten (insbesondere linearen) Fettalkohol mit 8 bis 22 Kohlenstoffatomen. Es handelt sich dabei ganz besonders bevorzugt um Ceteareth-15, Ceteareth-25 oder Ceteareth-50, welche als Eumulgin® CS 15 (COGNIS), Cremophor A25 (BASF SE) bzw. Eumulgin® CS 50 (COGNIS) vermarktet werden.

Die erfindungsgemäß verwendeten Mittel können weiterhin die Hilfs- und Zusatzstoffe enthalten, die üblicherweise herkömmlichen Stylingmitteln zugesetzt werden.

Als geeignete Hilfs- und Zusatzstoffe sind insbesondere zusätzliche Pflegestoffe zu nennen.

Als Pflegestoff kann beispielsweise ein Silikonöl und/oder ein Silikongum eingesetzt werden.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.
Dimethicone bilden die zweite Gruppe der Silikone, welche erfindungsgemäß enthalten sein können. Diese können sowohl linear als auch verzweigt als auch cyclisch oder cyclisch und verzweigt sein. Dimethiconcopolyole bilden eine weitere Gruppe von Silikonen, die geeignet sind. Besonders geeignete Silikone sind aminofunktionelle Silikone, insbesondere die Silikone, die unter der INCI-Bezeichnung Amodimethicone zusammengefasst sind.
Die Mittel enthalten die Silikone bevorzugt in Mengen von 0,01 Gew.-% bis 15 Gew.-%, besonders bevorzugt von 0,05 bis 2 Gew.-%, bezogen auf das gesamte Mittel.

Als Pflegestoff einer anderen Verbindungsklasse kann das Mittel beispielsweise mindestens ein Proteinhydrolysat und/oder eines seiner Derivate enthalten. Die Proteinhydrolysate sind in den erfindungsgemäßen Mitteln beispielsweise in Konzentrationen von 0,01 Gew.-% bis zu 20 Gew.-%, vorzugsweise von 0,05 Gew.-% bis zu 15 Gew.-% und ganz besonders bevorzugt in Mengen von 0,05 Gew.-% bis zu 5 Gew.-%, jeweils bezogen auf die gesamte Anwendungszubereitung enthalten.
Als Pflegestoff kann das erfindungsgemäße Mittel weiterhin mindestens ein Vitamin, ein Provitamin, eine Vitaminvorstufe und/oder eines derer Derivate enthalten. Ganz besonders bevorzugt wird als Pflegestoff D-Panthenol, gegebenenfalls in Kombination mit mindestens einem der oben genannten Silikonderivate eingesetzt.
Als Pflegestoff können die erfindungsgemäßen Mittel weiterhin mindestens einen Pflanzenextrakt enthalten
Auch Mono- bzw. Oligosaccharide können als Pflegestoff in den erfindungsgemäßen Mitteln eingesetzt werden. Die Mono- bzw. Oligosaccharide sind in den erfindungsgemäßen Mitteln bevorzugt in einer Menge von 0,1 bis 8 Gew.-%, insbesondere bevorzugt 1 bis 5 Gew.-%, bezogen auf die gesamte Anwendungszubereitung, enthalten.
Das Mittel kann weiterhin mindestens ein Lipid als Pflegestoff enthalten. Die erfindungsgemäßen Mittel enthalten die Lipide bevorzugt in Mengen von 0,01 bis 10 Gew.-%, insbesondere 0,1 bis 5 Gew.-%, bezogen auf die gesamte Anwendungszubereitung.

Weiterhin sind als Pflegestoff Ölkörper geeignet.

Zu den natürlichen und synthetischen kosmetischen Ölkörpern sind beispielsweise zu zählen:
- pflanzliche Öle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether.
- Esteröle. Unter Esterölen sind zu verstehen die Ester von C₆ - C₃₀ - Fettsäuren mit C₂ - C₃₀ - Fettalkoholen.
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I), 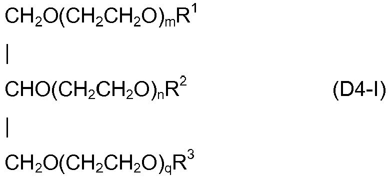
in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethyl-hexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäßen Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Obwohl jeder der genannten Pflegestoffe für sich alleine bereits ein zufrieden stellendes Resultat ergibt, sind im Rahmen der vorliegenden Erfindung auch alle Ausführungsformen umfasst, in denen das Mittel mehrere Pflegestoffe auch aus verschiedenen Gruppen enthält.

Durch Zugabe eines UV-Filters können sowohl die Mittel selbst, als auch die behandelten Fasern vor schädlichen Einflüssen von UV-Strahlung geschützt werden. Die UV-Filter sind üblicherweise in Mena¬gen von 0,01-5 Gew.-%, bezogen auf die gesamte Anwendungszubereitung, enthalten. Mengen von 0,1-2,5 Gew.-% sind bevorzugt.

In einer besonderen Ausführungsform enthält das erfindungsgemäße Mittel weiterhin einen oder mehrere direktziehende Farbstoffe. Dies ermöglicht, dass bei Anwendung des Mittels die behandelte keratinische Faser nicht nur temporär strukturiert, sondern zugleich auch gefärbt wird. Das kann insbesondere dann wünschenswert sein, wenn nur eine temporäre Färbung beispielsweise mit auffälligen Modefarben gewünscht wird, die sich durch einfaches Waschen wieder aus der keratinischen Faser entfernen lässt.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 11, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol. Bevorzugt werden kationische direktziehende Farbstoffe eingesetzt. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.
Die Farbstoffe, die auch unter den Bezeichnungen Basic Yellow 87, Basic Orange 31 und Basic Red 51 bekannt sind, sind ganz besonders bevorzugte kationische direktziehende Farbstoffe der Gruppe (c). Die kationischen direktziehenden Farbstoffe, die unter dem Warenzeichen Arianor® vertrieben werden, sind erfindungsgemäß ebenfalls ganz besonders bevorzugte kationische direktziehende Farbstoffe.
Die erfindungsgemäßen Mittel gemäß dieser Ausführungsform enthalten die direktziehenden Farbstoffe bevorzugt in einer Menge von 0,001 bis 20 Gew.-%, bezogen auf das gesamte Mittel.

Es ist erfindungsgemäß bevorzugt, dass die erfindungsgemäßen Mittel frei von Oxidationsfarbstoffvorprodukten sind. Oxidationsfarbstoffvorprodukte werden eingeteilt in sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Die Formulierung der erfindungsgemäßen Mittel kann in allen für Stylingmittel üblichen Formen erfolgen, beispielsweise in Form von Lösungen, die als Haarwasser oder Pump- oder Aerosolspray auf das Haar aufgebracht werden können, in Form von Cremes, Emulsionen, Wachsen, Gelen oder auch tensidhaltigen schäumenden Lösungen oder anderen Zubereitungen, die für die Anwendung auf dem Haar geeignet sind.

Ein zweiter Gegenstand der Erfindung ist ein Verfahren zur Umformung, insbesondere zur Glättung, keratinhaltiger Fasern, insbesondere menschlicher Haare, worin
(i) die Fasern einer Wärmebehandlung unterzogen werden,
(ii) die wärmebehandelten Fasern im Anschluß an die Wärmebehandlung mit einem kosmetischen Mittel nachbehandelt werden, das in einem kosmetischen Träger mindestens eine Verbindung der Formel (I) enthält, 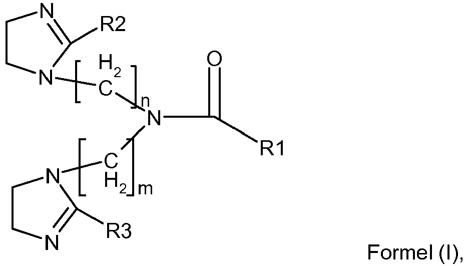 in welcher
   - R1, R2 und R3 unabhängig voneinander stehen für einen aliphatischen Kohlenwasserstoffrest mit 6 bis 30 Kohlenstoffatomen, und
   - n und m stehen unabhängig voneinander für ganze Zahlen von 1 bis 10,
(iii) und die Fasern unter Zuhilfenahme von Verformungshilfsmitteln nach, vor oder während Schritt (i) verformt, werden.

Es gelten die im Rahmen des ersten Erfindungsgegenstandes beschriebenen bevorzugten Ausführungsformen der Verbindungen der Formel (I) sowie die darin beschriebenen bevorzugten Ausführungsformen des besagten kosmetischen Mittels *mutatis mutandis* als bevorzugt.

Verformungshilfsmittel im Sinne des erfindungsgemäßen Verfahrens können
- z.B. Lockenwickler oder Papilloten im Falle einer Dauerwelle,
- ein Heißluftgebläse, wie ein Föhn oder ein Lockenstab,
- ein Welleisen,
- oder Hilfsmittel für eine mechanische Glättung, wie ein Kamm oder eine Bürste, ein Glättungsboard oder ein beheizbares Glätteisen im Falle einer Haarglättung sein.

Ganz besonders bevorzugt wird als Verformungshilfsmittel zumindest ein beheizbares Glätteisen eingesetzt.

Unter einer mechanischen Glättung wird erfindungsgemäß eine Streckung der krausen Faser entlang ihrer längsten räumlichen Ausdehnung verstanden.

Es ist bevorzugt, im Rahmen einer Haarglättung die Fasern mechanisch, insbesondere durch Kämmen oder mit Hilfe eines Glättungsboardes, zu glätten.

Wenn die Verformungshilfsmittel, beispielsweise Wickler, im Rahmen eines Wellverfahrens für einen längeren Zeitraum an der Faser befestigt werden, so ist es zweckmäßig, diese Verformungshilfsmittel spätestens nach Schritt (ii) zu entfernen.

In einer bevorzugten Ausführungsform der Erfindung werden die keratinhaltigen Fasern vor dem Schritt (i) angefeuchtet. Dies kann durch Besprühen der Fasern mit einer Flüssigkeit, bevorzugt mit Wasser, geschehen. Bevorzugterweise werden die Fasern vor Schritt (i) mit einem herkömmlichen Shampoo shampooniert, gespült und dann mit einem Handtuch frottiert. Nach Abschluß des Frottierschritts bleibt eine fühlbare Restfeuchtigkeit im Haar zurück. Die Fasern können zusätzlich mit einem kosmetischen Mittel benetzt werden, das die Wärmebehandlung unterstützt, z.B. das Gleiten von beheizten Glätteisen entlang der Faser während der Wärmebehandlung im Schritt (i) erleichtert. Geeignete kosmetische Mittel werden beispielsweise in der Druckschrift WO-A-2005/115319 beschrieben.

Es ist erfindungsgemäß bevorzugt, die Fasern vor der Wärmebehandlung des Schritts (i) zu trocknen. Eine trockene keratinhaltige Faser gemäß Schritt (ii) des erfindungsgemäßen Verfahrens liegt dann vor, wenn die den Haaren anhaftenden Wasserreste soweit verdampft sind, dass die Haare einzeln fallen. Bevorzugt ist bei einer trockenen keratinhaltigen Faser entweder der Feuchtigkeitsgehalt der Faser mit der Feuchtigkeit der Luft im wesentlichen im Gleichgewicht oder die Faser nimmt Feuchtigkeit aus der Luft der Umgebung auf. Eine solche trockene Faser wird bevorzugt durch Trocknung der nassen Faser mit heißer Luft unter Benutzung eines Föns erzielt. Die Trocknung vor Schritt (i) wird bevorzugt dann ausgeführt, wenn im Schritt (i) im Rahmen eines Umformungsverfahrens eine Wärmebehandlung erfolgt, in dem die Oberfläche der keratinhaltigen Fasern direkt mit einer temperierten Oberfläche einer Verformungshilfe (z.B. Glätteisen oder Welleisen) während eines Umformungsschritts erfolgt.

In einer bevorzugten Ausführungsform werden die Fasern im Schritt (i) einer Wärmebehandlung unter gleichzeitiger Verformung der Faser bei einer Temperatur von 50°C bis 350°C (vorzugsweise 80°C bis 280°C, besonders bevorzugt 100°C bis 250°C, weiter bevorzugt 140°C bis 220°C) unterworfen. Gleichzeitige Verformung bedeutet, dass die Faser während der Wärmebehandlung in eine Form, z.B. durch mechanische Glättung oder Festlegung der Form durch Wickler oder Papilloten, gebracht oder gehalten werden.

In einer speziellen Ausführungsform werden im Rahmen einer Haarglättung die Fasern in Schritt (i) einer Wärmebehandlung unter mechanischer Glättung der Faser bei einer Temperatur von 50°C bis 350°C (vorzugsweise 80°C bis 280°C, besonders bevorzugt 100°C bis 250°C, weiter bevorzugt 140°C bis 220°C) unterworfen. Die Wärmebehandlung kann mit heißer Luft erfolgen. In diesem Fall, wird die Faser während des Kämmens genau an der Stelle erwärmt, an der die mechanische Glättung erfolgt. Darüber hinaus ist es besonders bevorzugt, dass die Wärmebehandlung nach Manier des Glättens mit Hilfe eines sogenannten Glätteisens mit entsprechend temperierter Platten, insbesondere Metall- oder Keramikplatten, erfolgt, in dem die Platte auf die zu glättende Faser gedrückt wird und die an die Faser gedrückte Platte entlang der Faser bewegt wird. Die Platten können gegebenenfalls mit hitzebeständigen Werkstoffen beschichtet sein. Besonders bevorzugt wird die zu glättende keratinhaltige Faser zwischen zwei entsprechend temperierte Platten gepreßt und beide Platten zugleich entlang der längsten räumlichen Ausdehnung der Faser bewegt. Dabei ist es wiederum bevorzugt, dass beide Platten miteinander verbunden sind, so dass beide Platten gleichmäßig entlang der Faser bewegt werden können. Wird die Wärmebehandlung am lebenden Haar durchgeführt, so ist die Faser an einem Ende (Haarwurzel) befestigt. Die Platten werden in diesem Fall bevorzugt gleichmäßig von der Haarwurzel weg entlang der gesamten Faser bewegt. Durch diese Bewegung erfolgt eine mechanische Glättung der Faser. Ein entsprechendes Gerät zur Wärmebehandlung ist beispielsweise das Gerät "Ceramic Flat-Master" (Vertrieben durch: Efalock, Deutschland) oder Schwarzkopf Professional: Modell-Nr: IP30A.

In einer weiteren Ausführungsform werden im Rahmen einer Wellung die Fasern in einem Schritt (i) einer Wärmebehandlung unter mechanischer Krausung der Faser bei einer Temperatur von 50°C bis 350°C (vorzugsweise 80°C bis 280°C, besonders bevorzugt 100°C bis 250°C, weiter bevorzugt 140°C bis 220°C) unterworfen. Die Wärmebehandlung kann mit einem Heißluftgebläse erfolgen. In diesem Fall, wird entweder die Faser um einen entsprechenen Heißluftlockenstab gewickelt und genau an der Stelle erwärmt, an der die Krausung erfolgt. Oder die Fasern werden auf Lockenwickler als Verformungshilfe gewickelt und mit einem Fön erwärmt. Eine weitere Möglichkeit der wärmeunterstützten Wellung erfolgt, in dem die Fasern ohne Einsatz eines Heißluftgebläses um einen Stab mit entsprechend aufgeheizter Oberfläche gewickelt und genau an der Stelle erwärmt, an der die Krausung erfolgt. Ein entsprechendes Gerät zur Wärmebehandlung ist beispielsweise das Gerät von Schwarzkopf Professional, Modell-Nr: CPL Type F37.

Eine besonders bevorzugte Ausführungsform der vorliegenden Erfindung wird durch die folgenden Punkte definiert:
1. Verwendung von mindestens einer Verbindung der Formel (I) 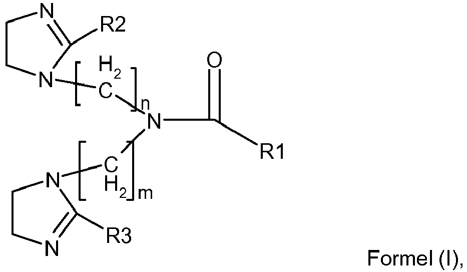 in welcher
   - R1, R2 und R3 unabhängig voneinander stehen für einen aliphatischen Kohlenwasserstoffrest mit 6 bis 30 Kohlenstoffatomen, und
   - n und m stehen unabhängig voneinander für ganze Zahlen von 1 bis 10, in einem Nachbehandlungsschritt nach der Anwendung von Wärme zur Restrukturierung keratinhaltiger Fasern, die Wärme von einer Temperatur von 80°C bis 350°C (vorzugsweise 80°C bis 280°C, besonders bevorzugt 100°C bis 250°C, weiter bevorzugt 140°C bis 220°C) ausgesetzt wurden.
2. Verwendung gemäß Punkt 1, dadurch gekennzeichnet, dass die Wärmequelle ein Glätteisen oder Welleisen ist.
3. Verwendung nach einem der Punkte 1 oder 2, dadurch gekennzeichnet, dass besagter aliphatische Kohlenwasserstoffrest der Reste R1, R2 und R3 für eine C6-C30-Alkylgruppe oder eine C6-C30-Alkenylgruppe (besonders bevorzugt für eine C8-C24-Alkylgruppe oder eine C8-C24-Alkenylgruppe, ganz besonders bevorzugt für eine C10-C20-Alkylgruppe oder eine C10-C20-Alkenylgruppe, und höchst bevorzugt für eine C12-C18-Alkylgruppe oder eine C12-C18-Alkenylgruppe) steht.
4. Verwendung nach einem der Punkte 1 bis 3, dadurch gekennzeichnet, dass
5. Verwendung nach einem der Punkte 1 bis 4, dadurch gekennzeichnet, dass zumindest die Reste R2 und R3 der Formel (I) gleich sind.
6. Verwendung nach einem der Punkte 1 bis 5, dadurch gekennzeichnet, dass die Reste R1 und R2 und R3 der Formel (I) gleich sind.
7. Verwendung nach einem der Punkte 1 bis 6, dadurch gekennzeichnet, dass gemäß Formel (I) n = m ist.
8. Verwendung nach einem der Punkte 1 bis 7, dadurch gekennzeichnet, dass n und m der Formel (I) für ganze Zahlen von 2 bis 6 stehen und höchst bevorzugt für 2, 3 und/oder 4.
9. Verfahren zur Umformung, insbesondere zur Glättung, keratinhaltiger Fasern, insbesondere menschlicher Haare, worin
   (i) die Fasern einer Wärmebehandlung unterzogen werden,
   (ii) die wärmebehandelten Fasern im Anschluß an die Wärmebehandlung mit einem kosmetischen Mittel nachbehandelt werden, das in einem kosmetischen Träger mindestens eine Verbindung der Formel (I) enthält, 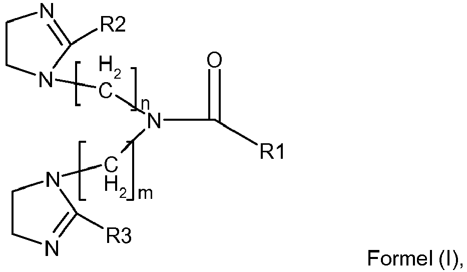 in welcher
      - R1, R2 und R3 unabhängig voneinander stehen für einen aliphatischen Kohlenwasserstoffrest mit 6 bis 30 Kohlenstoffatomen, und
      - n und m stehen unabhängig voneinander für ganze Zahlen von 1 bis 10,
   (iii) und die Fasern unter Zuhilfenahme von Verformungshilfsmitteln nach, vor oder während Schritt (i) verformt, werden.
10. Verfahren nach Punkt 9, dadurch gekennzeichnet, dass in Schritt (i) einer Wärmebehandlung unter gleichzeitiger Verformung der Faser bei einer Temperatur von 50°C bis 350°C (vorzugsweise 80°C bis 280°C, besonders bevorzugt 100°C bis 250°C, weiter bevorzugt 140°C bis 220°C) unterworfen werden.
11. Verfahren nach einem der Punkte 9 oder 10, dadurch gekennzeichnet, dass als Verformungshilfsmittel zumindest ein beheizbares Glätteisen eingesetzt wird.
12. Verfahren nach einem der Punkte 9 bis 11, dadurch gekennzeichnet, dass die Verbindung der Formel (I) wie in einem der Punkte 3 bis 8 definiert wird.
13. Verfahren nach einem der Punkte 9 bis 12, dadurch gekennzeichnet, dass die Verbindungen der obigen Formel (I) in einer Menge von 0,01 bis 15,0 Gew.-%, besonders bevorzugt von 0,1 bis 10,0 Gew.-%, ganz besonders bevorzugt von 0,1 bis 7,5 Gew.-%, höchst bevorzugt von 0,3 bis 5,0 Gew.% jeweils bezogen auf das Gewicht des anwendungsbereiten Mittels, enthalten sind.

### Beispiele

Alle Mengenangaben sind in Gew.-% bezogen auf das Gesamtgewicht des Mittel, falls nicht anders gekennzeichnet.

### 1.0 Beispielrezeptur

Es wurde folgendes erfindungsgemäßes kosmetisches Mittel durch mischen der Komponenten hergestellt.

**Tabelle 1:**

| Rohstoff | Menge |
|---|---|
| Luviskol VA 64 W ¹ | 5,00 |
| Gluadin WQ ² | 1,00 |
| Keradyn HH-LQ-(RB) ³ | 1,00 |
| Natrium Benzoat | 0,10 |
| Cetyltrimethylammoniumchlorid | 0,50 |
| ortho-Phosphorsäure | 0,024 |
| Hydrolyzed Silk Protein | 0,50 |
| PEG-40 Hydrogenated Castor Oil | 2,50 |
| Ethanol | 5,00 |
| Wasser (destilliert) | ad 100 |

| | |
|---|---|
| ¹ Copolymer aus Vinylpyrrolidon und Vinylacetat (60:40) (48-52% Aktivsubstanz in Wasser, INCI-Bezeichnung: VP/VA Copolymer) (BASF) ² Weizenproteinhydrolysat (ca. 31-35% Festkörper; INCI-Bezeichnung: Aqua (Water), Laurdimonium Hydroxypropyl Hydrolyzed Wheat Protein, Ethylparaben, Methylparaben) (BASF SE) ³ Bis-Ethyl(isostearylimidazoline) Isostearamide (Croda) | |

### 2.0 Wirknachweis:

Für den Wirknachweis wurde obige Zusammensetzung eingesetzt.

### 2.1 Prüfmethodik HP-DSC (High Pressure Differential Scanning Calorimetry)

Thermoanalytische Untersuchungen eignen sich besonders zur Charakterisierung von Zweiphasensystemen, zu denen die Humanhaare als Faserkeratine mit ihrem kristallinen α-Helix-Anteil und amorphen Matrix-Anteil ebenfalls gehören. Auf der einen Seite können Glasübergänge und Alterungsverhalten der amorphen Matrix untersucht werden, auf der anderen Seite liefert das Schmelzverhalten der kristallinen, helicalen Phase wichtige Erkenntnisse. Thermoanalytische Untersuchungen sind erstmals 1899 beschrieben, erste Differenzthermoanalysen (DTA) an Proteinfasern wurden Ende der fünfziger Jahre durchgeführt. In den folgenden Jahren sind unterschiedliche thermoanalytische Messverfahren, wie DTA, HP-DTA (High Pressure, Hochdruck-DTA) und DSC (Differential Scanning Calorimetry, Dynamische Differenz-Kalorimetrie), an Keratinfasern angewendet worden um z.B. das Phänomen der Superkontraktion, α-β-Phasenübergänge der Helices oder Denaturierungsvorgänge zu untersuchen. Es wurde die Methode der HP-DSC zur Untersuchung von Keratinfasern genutzt, die die Probleme mit pyrolytischen Effekten, wie sie bei der konventionellen DSC auftreten, und Probleme mit der Datenerfassung und -interpretation, wie sie die DTA birgt, ausschließt. Dabei werden DSC-Messungen an Keratinen durchgeführt, die mit Wasser in kommerziell erhältlichen, druckfesten Meßkapseln eingeschlossen sind. Im Keratin-Wasser-System entwickelt sich beim Erhitzen oberhalb von 100°C in den verkapselten Stahltiegeln ein Wasserdampfhochdruck, woraus sich die HP-DSC Analyse ableitet. Der gravierende Unterschied der HP-DSC-Thermogramme von Humanhaaren im Vergleich zu normalen DSC-Thermogrammen ist der, dass die endothermen Peaks, die den Umwandlungspunkt und Umwandlungsenthalpie wiedergeben, hier um ca. 90 °C zu niedrigeren Temperaturen verschoben sind. Das rührt daher, dass das Wasser nach Diffusion in die Haarfaser durch Schwächung und Spaltung von Wasserstoffbrücken- und Salzbindungen die Proteinstabilität vermindert und so die "Verleimungstemperatur" der Keratine herabsetzt. Werden durch das superkontrahierende Agens wie Wasser nur Wasserstoffbrücken und Salzbrücken gelöst, so ist der thermische Effekt reversibel (Superkontraktion). Der Vorgang wird jedoch irreversibel, sobald auch kovalente Bindungen, wie z. B. Disulfidbrücken, gespalten werden. Dies tritt ein, wenn man Humanhaarfasern in druckfesten Kapseln mit Wasser auf über 150 °C erhitzt. Die irreversible Umwandlung, interpretiert als Übergang der α-helicalen Bereiche in den Proteinen in einen ungeordneten Zustand, resultiert in endothermen Peaks, wobei die Peaklage den Umwandlungs- oder auch Denaturierungspunkt und die Peakfläche die Umwandlungs- oder Denaturierungs-Enthalpie wiedergibt.

Unter Verwendung der Dynamischen Differenz-Kalorimetrie (DSC) können demnach strukturelle sowie chemische Zustände und Veränderungen in Faserkeratinen und insbesondere in Humanhaaren erfaßt werden. Unter genau definierten Versuchsbedingungen kann man bei Humanhaaren die kalorimetrisch erfassbaren Vorgänge anhand von Thermogrammen aufzeichnen und sie bezüglich der Peaklagen, -strukturen und -flächen als Indikator für die Beeinflussung von Ordnungs-Unordnungsübergangen durch Änderungen innerer und/oder äußerer Parameter, hervorrufen z. B. durch kosmetische Behandlung der Haare, verwenden. D. h. aus den im Thermogramm von Humanhaaren aufgezeichneten endothermen Peaks lassen sich aufgrund von Peaklage (Umwandlungspunkt) und Peakfläche (Umwandlungsenthalpie) Aussagen über Festigkeit bzw. Schädigung der Humanhaarfaser treffen.

Ausführliche Untersuchungen bezüglich des Einflusses des Cystingehaltes auf die Denaturierung der α-Helices in Keratinen haben z. B. gezeigt, dass die Denaturierungs-Temperatur (Übergangstemperatur) des Keratins linear mit dem Cystingehalt ansteigt. Die erhöhte Stabilität des Matrixbereichs aufgrund des höheren Vernetzungsgrades des erhöhten Anteils an Disulfidbrücken in der Matrix führt dazu, dass die Umwandlung der in diese Matrix eingebetteten Helices erschwert wird und resultiert somit in einer Erhöhung der Denaturierungs-Temperatur. Umgekehrt kann in der Regel eine Denaturierungs-Temperatur- und vor allem Denaturierungs-Enthalpieerniedrigung bei durch Dauerwelle oder Bleichung bzw. Färbung behandelten Humanhaaren beobachtet werden (H. Deutz, Doktorarbeit, RWTH Aachen 1993).

### 2.2 Durchführung

### 2.2.1 Variante des Verfahrens "Verwendung der Verbindung nach Formel (I) während der Glättung am Haar"

Humanhaarsträhnen (Kerling International (Backnang, Deutschland) "European natural hair 7/0") wurden jeweils 1,8 g Strähne mit 0,7 g einer der in Tabelle 1 aufgelisteten Testzusammensetzungen behandelt und ohne Spülschritt an der Luft getrocknet.

Es wurde ein Glätteisen (Marke: Schwarzkopf Professional; Modell-Nr: IP30A) auf höchster Stufe (210°C) für mindestens 10 Minuten aufgeheizt. Die mit einem Kontaktthermometer (Marke: Qtemp 300) gemessene Temperatur zwischen den Heizplatten betrug nach 10min 180°C.

Nun wurde die getrocknete Haarsträhne mit dem vorgeheizten Glätteisen geglättet. Dabei wurde auf folgende Führung des Glätteisens entlang der Haarfaser geachtet: 4 mal kurz (je 1 Sekunde pro Haarsträhne), 2 mal lang (5 Sekunden pro Haarsträhne).
Die erreichten Temperaturen schwanken dabei zwischen 146 bis 181°C.

Die Haarsträhne wurde an der Luft auf Raumtemperatur abgekühlt, ca. 1 Minute lang mit Wasser gespült (Wassertemperatur ca. 35 bis 39°C), überschüssiges Wasser mit dem Finger entfernt und mit einem Handtuch trocken getupft, aufgehängt und ruhen und trocknen gelassen.

Diese Behandlung wurde für jede Strähne 40 Mal wiederholt.

### 2.2.2 Variante des Verfahrens "Verwendung der Verbindung der Formel (I) als Nachbehandlungsmittel nach der Glättung"

Es wurden Humanhaarsträhnen (Kerling International (Backnang, Deutschland) "European natural hair 7/0") verwendet. Es wurde ein Glätteisen (Marke: Schwarzkopf Professional; Modell-Nr: IP30A) auf höchster Stufe (210°C) für mindestens 10 Minuten aufgeheizt. Die mit einem Kontaktthermometer (Marke: Qtemp 300) gemessene Temperatur zwischen den Heizplatten betrug nach 10min 180°C.

Nun wurde die Haarsträhne mit dem vorgeheizten Glätteisen geglättet. Dabei wurde auf folgende Führung des Glätteisens entlang der Haarfaser geachtet: 4 mal kurz (je 1 Sekunde pro Haarsträhne), 2 mal lang (5 Sekunden pro Haarsträhne).
Die erreichten Temperaturen schwanken dabei zwischen 146 bis 181 °C.

Die Haarsträhne wurde an der Luft auf Raumtemperatur abgekühlt. Dann wurde das obige Mittel auf die Faser aufgetragen und über Nacht auf der Faser belassen.

Sodann wurde die Strähne ca. 1 Minute lang mit Wasser gespült (Wassertemperatur ca. 35 bis 39°C), überschüssiges Wasser mit dem Finger entfernt und mit einem Handtuch trocken getupft, aufgehängt und ruhen und trocknen gelassen.

Diese Behandlung wurde für jede Strähne 40 Mal wiederholt.

### 2.3 thermoanalytische Untersuchung

Mittels thermoanalytischer Untersuchungen (DSC-Gerät: Perkin Elmer DSC 7, 60 µl Edelstahlmesstiegel mit O-Ring (24 atm)) wurde dann auf Strukturstabilisierung durch die Testzusammensetzung nach der wiederholten Hitzeanwendung, i.e. nach Abschluss der jeweiligen Verfahren aus 2.2.1 und 2.2.2 geprüft. Dabei wurde jede Strähne in Teile zu je ca. 1 mm geteilt. 12 dieser aliquoten Teile derselben Strähne wurden in einen DSC-Messtiegel gefüllt und 50 µl bidestilliertes Wasser gegeben. Die Messtiegel wurden verschlossen und die Probe in einem Temperaturintervall von 100°C bis 170°C (Heizrate: 10 Kelvin pro Minute) vermessen.

Auch das unbehandelte Ausgangshaar wurde ebenso einer HP-DSC-Messung unterzogen.

Je Verfahrensvariante wurden 5 Strähnen getestet. Die durch arithmetisches Mittel aus den Einzelmessungen berechneten Keratinschmelzpunkte sind in Tabelle 2 aufgelistet.

**Tabelle 2: Hitzeschutz der Struktur durch die erfindungsgemäße Wirkstoffkombination**

| **Verfahren** | **Keratinschmelzpunkt [°C]** |
|---|---|
| während Hitzeanwendung | 131.6 |
| **Nachbehandlung** | **136**.**3** |
| unbehandeltes Ausgangshaar | 155.2 |

Der Keratinschmelzpunkt der mit Verbindungen der Formel (I) nachbehandelten Strähnen ist signifikant höher als bei den Strähnen, die mit Verbindungen der Formel (I) währen der Hitzeanwendung ausgestattet waren. Durch die Nachbehandlung wird folglich die Haarstruktur vor der Hitzeeinwirkung wirkungsvoll geschützt.

## Patentansprüche

1. Verwendung von mindestens einer Verbindung der Formel (I) 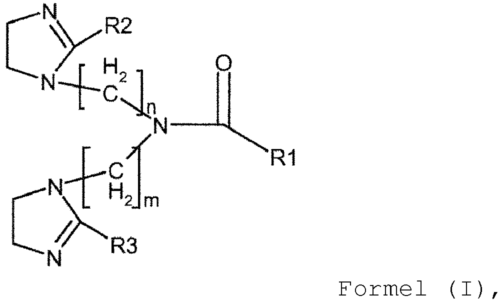 in welcher
- R1, R2 und R3 unabhängig voneinander für einen aliphatischen Kohlenwasserstoffrest mit 6 bis 30 Kohlenstoffatomen stehen, und
- n und m unabhängig voneinander für ganze Zahlen von 1 bis 10 stehen,
in einem Nachbehandlungsschritt nach der Anwendung von Wärme zur Restrukturierung keratinhaltiger Fasern, die Wärme von einer Temperatur von 80°C bis 350°C (vorzugsweise 80°C bis 280°C, besonders bevorzugt 100°C bis 250°C, weiter bevorzugt 140°C bis 220°C) ausgesetzt wurden.

2. Verwendung nach Anspruch 1, **dadurch gekennzeichnet, dass** die Wärmequelle ein Glätteisen oder Welleisen ist.

3. Verwendung nach einem der Ansprüche 1 oder 2, **dadurch gekennzeichnet, dass** besagter aliphatischer Kohlenwasserstoffrest der Reste R1, R2 und R3 für eine C6-C30-Alkylgruppe oder eine C6-C30-Alkenylgruppe (besonders bevorzugt für eine C8-C24-Alkylgruppe oder eine C8-C24-Alkenylgruppe, ganz besonders bevorzugt für eine C10-C20-Alkylgruppe oder eine C10-C20-Alkenylgruppe, und höchst bevorzugt für eine C12-C18-Alkylgruppe oder eine C12-C18-Alkenylgruppe) steht.

4. Verwendung nach einem der Ansprüche 1 bis 3, **dadurch gekennzeichnet, dass** zumindest die Reste R2 und R3 der Formel (I) gleich sind.

5. Verwendung nach einem der Ansprüche 1 bis 4, **dadurch gekennzeichnet, dass** die Reste R1 und R2 und R3 der Formel (I) gleich sind.

6. Verwendung nach einem der Ansprüche 1 bis 5, **dadurch gekennzeichnet, dass** gemäß Formel (I) n = m ist.

7. Verwendung nach einem der Ansprüche 1 bis 6, **dadurch gekennzeichnet, dass** n und m der Formel (I) für ganze Zahlen von 2 bis 6 stehen und höchst bevorzugt für 2, 3 und/oder 4.

8. Verfahren zur Umformung, insbesondere zur Glättung, keratinhaltiger Fasern, insbesondere menschlicher Haare, worin
(i) die Fasern einer Wärmebehandlung unterzogen werden,
(ii) die wärmebehandelten Fasern im Anschluss an die Wärmebehandlung nach der Anwendung von Wärme mit einem kosmetischen Mittel nachbehandelt werden, das in einem kosmetischen Träger mindestens eine Verbindung der Formel (I) enthält, 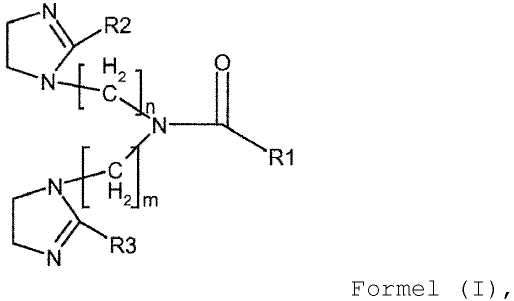 in welcher
- R1, R2 und R3 unabhängig voneinander für einen aliphatischen Kohlenwasserstoffrest mit 6 bis 30 Kohlenstoffatomen stehen, und
- n und m unabhängig voneinander für ganze Zahlen von 1 bis 10 stehen,
(iii) und die Fasern unter Zuhilfenahme von Verformungshilfsmitteln nach, vor oder während Schritt (i) verformt werden.

9. Verfahren nach Anspruch 8, **dadurch gekennzeichnet, dass** in Schritt (i) die Fasern einer Wärmebehandlung unter gleichzeitiger Verformung der Fasern bei einer Temperatur von 50°C bis 350°C (vorzugsweise 80°C bis 280°C, besonders bevorzugt 100°C bis 250°C, weiter bevorzugt 140°C bis 220°C) unterworfen werden.

10. Verfahren nach einem der Ansprüche 8 oder 9, **dadurch gekennzeichnet, dass** als Verformungshilfsmittel zumindest ein beheizbares Glätteisen eingesetzt wird.

11. Verfahren nach einem der Ansprüche 8 bis 10, **dadurch gekennzeichnet, dass** die Verbindung der Formel (I) wie in einem der Punkte 3 bis 8 definiert wird.

12. Verfahren nach einem der Ansprüche 8 bis 11, **dadurch gekennzeichnet, dass** die Verbindungen der obigen Formel (I) in einer Menge von 0,01 bis 15,0 Gew.-%, besonders bevorzugt von 0,1 bis 10,0 Gew.-%, ganz besonders bevorzugt von 0,1 bis 7,5 Gew.-%, höchst bevorzugt von 0,3 bis 5,0 Gew.% jeweils bezogen auf das Gewicht des anwendungsbereiten Mittels, enthalten sind.

## Claims

1. Use of at least one compound of the formula (I) 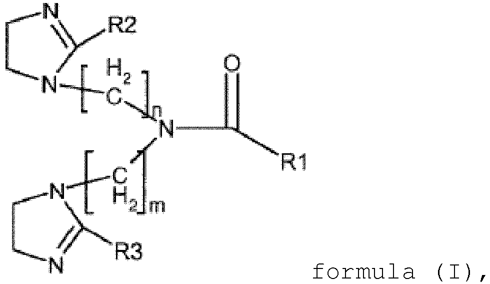 in which
- R1, R2 and R3 are independently an aliphatic hydrocarbyl radical having 6 to 30 carbon atoms, and
- n and m are independently integers from 1 to 10,
in a post-treatment step after the application of heat for restructuring keratin fibres which have been exposed to heat of a temperature of 80°C to 350°C (preferably 80°C to 280°C, particularly preferably 100°C to 250°C, more preferably 140°C to 220°C).

2. Use according to Claim 1, **characterized in that** the heat source is a straightening iron or curling iron.

3. Use according to either of Claims 1 and 2, **characterized in that** said aliphatic hydrocarbyl radical of the radicals R1, R2 and R3 is a C6-C30 alkyl group or a C6-C30 alkenyl group (particularly preferably a C8-C24 alkyl group or a C8-C24 alkenyl group, very particularly preferably a C10-C20 alkyl group or a C10-C20 alkenyl group, and most preferably a C12-C18 alkyl group or a C12-C18 alkenyl group).

4. Use according to any of Claims 1 to 3, **characterized in that** at least the radicals R2 and R3 of the formula (I) are identical.

5. Use according to any of Claims 1 to 4, **characterized in that** the radicals R1 and R2 and R3 of the formula (I) are identical.

6. Use according to any of Claims 1 to 5, **characterized in that** n = m according to formula (I).

7. Use according to any of Claims 1 to 6, **characterized in that** n and m of the formula (I) are integers from 2 to 6 and are most preferably 2, 3 and/or 4.

8. Method for shaping, especially straightening, keratin fibres, especially human hairs, wherein
(i) the fibres are subjected to a heat treatment,
(ii) the heat-treated fibres are, following the heat treatment after the application of heat, post-treated with a cosmetic agent which contains in a cosmetic carrier at least one compound of the formula (I), 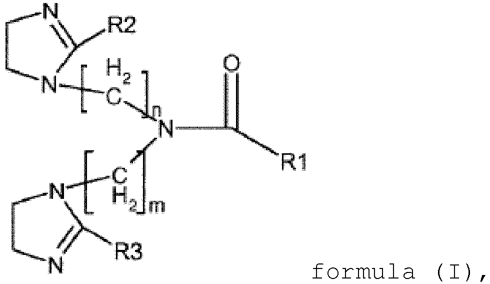 in which
- R1, R2 and R3 are independently an aliphatic hydrocarbyl radical having 6 to 30 carbon atoms, and
- n and m are independently integers from 1 to 10,
(iii) and the fibres are deformed with the aid of deformation aids after, before or during step (i).

9. Method according to Claim 8, **characterized in that**, in step (i), the fibres are subjected to a heat treatment with simultaneous deforming of the fibres at a temperature of 50°C to 350°C (preferably 80°C to 280°C, particularly preferably 100°C to 250°C, more preferably 140°C to 220°C).

10. Method according to either of Claims 8 and 9, **characterized in that** at least one heatable straightening iron is used as deformation aid.

11. Method according to any of Claims 8 to 10, **characterized in that** the compound of the formula (I) is as defined in any of points 3 to 8.

12. Method according to any of Claims 8 to 11, **characterized in that** the compounds of the above formula (I) are present in an amount of from 0.01 to 15.0% by weight, particularly preferably from 0.1 to 10.0% by weight, very particularly preferably from 0.1 to 7.5% by weight, most preferably from 0.3 to 5.0% by weight, based in each case on the weight of the ready-to-use agent.

## Revendications

1. Utilisation d'au moins un composé de formule (I) 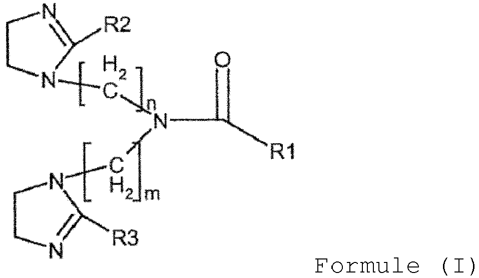 dans laquelle
- R1, R2 et R3 représentent indépendamment les uns des autres un radical hydrocarboné aliphatique de 6 à 30 atomes de carbone, et
- n et m représentent indépendamment l'un de l'autre des nombres entiers de 1 à 10,
dans une étape de post-traitement après l'application de chaleur pour la restructuration de fibres kératiniques qui ont été exposées à une chaleur d'une température allant de 80 °C à 350 °C (de préférence de 80 °C à 280 °C, de manière particulièrement préférée de 100 °C à 250 °C, de manière davantage préférée de 140 °C à 220 °C).

2. Utilisation selon la revendication 1, **caractérisée en ce que** la source de chaleur est un fer à lisser ou un fer à friser.

3. Utilisation selon l'une quelconque des revendications 1 ou 2, **caractérisée en ce que** ledit radical hydrocarboné aliphatique des radicaux R1, R2 et R3 représente un groupe alkyle en C6-C30 ou un groupe alcényle en C6-C30 (de manière particulièrement préférée un groupe alkyle en C8-C24 ou un groupe alcényle en C8-C24, de manière tout particulièrement préférée un groupe alkyle en C10-C20 ou un groupe alcényle en C10-C20, et de manière préférée entre toutes un groupe alkyle en C12-C18 ou un groupe alcényle en C12-C18).

4. Utilisation selon l'une quelconque des revendications 1 à 3, **caractérisée en ce qu'**au moins les radicaux R2 et R3 sont de formule (I).

5. Utilisation selon l'une quelconque des revendications 1 à 4, **caractérisée en ce que** les radicaux R1 et R2 et R3 sont de formule (I).

6. Utilisation selon l'une quelconque des revendications 1 à 5, **caractérisée en ce que**, dans la formule (I), n = m.

7. Utilisation selon l'une quelconque des revendications 1 à 6, **caractérisée en ce que** n et m de la formule (I) représentent des nombres entiers de 2 à 6 et de manière préférée entre toutes 2, 3 et/ou 4.

8. Procédé de façonnage, notamment de lissage, de fibres kératiniques, notamment de cheveux humains, selon lequel
(i) les fibres sont soumises à un traitement thermique,
(ii) immédiatement après le traitement thermique, les fibres traitées thermiquement sont post-traitées après l'application de chaleur avec un agent cosmétique, qui contient dans un véhicule cosmétique au moins un composé de formule (I), 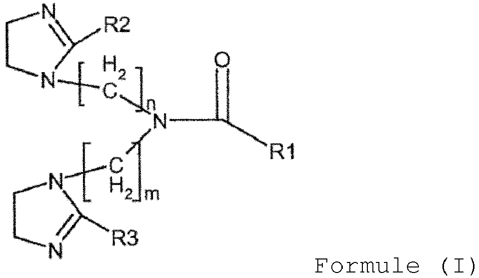 dans laquelle
- R1, R2 et R3 représentent indépendamment les uns des autres un radical hydrocarboné aliphatique de 6 à 30 atomes de carbone, et
- n et m représentent indépendamment l'un de l'autre des nombres entiers de 1 à 10,
(iii) et les fibres sont façonnées à l'aide d'auxiliaires de façonnage après, avant ou pendant l'étape (i).

9. Procédé selon la revendication 8, **caractérisé en ce qu'**à l'étape (i), les fibres sont soumises à un traitement thermique avec façonnage simultané des fibres à une température allant de 50 °C à 350 °C (de préférence de 80 °C à 280 °C, de manière particulièrement préférée de 100 °C à 250 °C, de manière davantage préférée de 140 °C à 220 °C).

10. Procédé selon l'une quelconque des revendications 8 ou 9, **caractérisé en ce qu'**au moins un fer à lisser chauffant est utilisé en tant qu'auxiliaire de façonnage.

11. Procédé selon l'une quelconque des revendications 8 à 10, **caractérisé en ce que** le composé de formule (I) est défini tel que dans l'un quelconque des points 3 à 8.

12. Procédé selon l'une quelconque des revendications 8 à 11, **caractérisé en ce que** les composés de la formule (I) ci-dessus sont contenus en une quantité de 0,01 à 15,0 % en poids, de manière particulièrement préférée de 0,1 à 10,0 % en poids, de manière tout particulièrement préférée de 0,1 à 7,5 % en poids, de manière préférée entre toutes de 0,3 à 5,0 % en poids, à chaque fois par rapport au poids de l'agent prêt à l'emploi.
